# EUROPEAN PATENT SPECIFICATION

(11) **EP 3 178 447 B1**
(45) Date of publication and mention of the grant of the patent: **18.03.2020**
(21) Application number: 16203566.1
(22) Date of filing: 12.12.2016
(51) Int. Cl.: A61F 2/40

(54) **OVAL HUMERAL HEADS**
OVALE HUMERUSKÖPFE
TÊTES HUMÉRALES OVALES

(30) Priority: 11.12.2015 US 201562266238 P
(43) Date of publication of application: 14.06.2017
(73) Proprietor: Zimmer, Inc., Warsaw, Indiana 46580 (US)
(72) Inventor: HOPKINS, Andrew, 8404 Winterthur (CH)
(74) Representative: Mays, Julie

(56) References cited:
- US-A- 4 261 062
- US-A- 4 919 670
- US-A1- 2007 225 818
- US-A1- 2009 118 837

## Description

### CLAIM OF PRIORITY

### TECHNICAL FIELD

This document pertains generally, but not by way of limitation, to an implant system for arthroplasty of shoulders and other joints.

### BACKGROUND

In the natural shoulder joint, the humeral head of the humerus is fitted to the glenoid cavity of the scapula. The shape of the natural humeral head approximates the shape of the glenoid cavity such that the humeral head maintains sufficient contact area between the humeral head and the glenoid cavity as the joint is articulated. Insufficient contact area can result in pain or discomfort during articulating of the joint. Insufficient contact area or an irregularly shaped contact area can cause irregular wear of the glenoid cavity, which can lead to other ailments. The natural glenoid cavity is typically non-spherically shaped making matching the humeral head to the glenoid cavity difficult. The natural glenoid cavity initially can be pear shaped, but can become worn into an oval shape over time. As such, the natural shape of the humeral head can be oval or non-spherically shaped to approximate the shape of the glenoid cavity.

Certain shoulder reconstructive surgeries, such as shoulder semi-arthroplasty, can involve replacing a portion of or an entire damaged humeral head with a humeral implant. Humeral implants are disclosed, for example, in US 2007/225818, US 4,261,062, US 2009/118837, and US 4,919,670. The humeral head implant is mounted to the humerus such that the simulated humeral head of the implant approximates the position of the natural humeral head. Following mounting of the humeral implant, the implant humeral head is fitted to the articulating surface of a natural glenoid cavity or an articulating surface of a glenoid implant that approximates the natural shape. The conventional humeral head is typically hemi-spherically shaped, which is easier to manufacture and provide standardized sizes. A drawback of the spherical shape can result in insufficient contact area between the humeral head and the glenoid cavity due to the incompatibility between the hemi-spherical humeral head and the non-spherical articulating surface.

### OVERVIEW

The present inventors have recognized, among other things, that a problem to be solved can include insufficient contact area between an implant humeral head improperly fit to an articulating surface of a natural glenoid cavity or a glenoid implant. In an example, the present subject matter can provide a solution to this problem, such as by providing a humeral implant having a non-spherical humeral head shaped to more closely approximate the articulating surface. The humeral head can include a main body having a curved exterior surface and a generally planar exterior surface, the humeral head defining a main body axis extending from the planar exterior surface to the curved exterior surface. The main body can define a polar axis oriented parallel to the generally planar surface and intersecting the main body axis. The main body also can define an equatorial axis transverse to the polar axis and oriented parallel to the generally planar surface, wherein the equatorial axis can intersect the main body axis at the polar axis. In an example, the equatorial axis can differ in length from the polar axis such that the humeral head is non-spherical.

In an example, the main body can define an edge transition between the curved exterior surface and the generally planar exterior surface. The edge transition can be positioned adjacent the outer edge of the main body or positioned inward of the outer edge of the main body such that the curved exterior surface curves inward. In at least one example, the distance of the edge transition from the main body axis along the polar axis can differ from the distance of the edge transition from the main body axis along the equatorial axis such that the curvature of the curved exterior surface varies around the exterior edge of the main body.

In an example, the main body can define a port for mounting the humeral head to a humeral stem mountable to a humerus. The humeral implant can include an attachment assembly engaging the port to the humeral stem. In at least one example, the port can be offset from the main body axis allowing the humeral head to be positioned offset from the humeral stem and correspondingly the humerus. The offset positioning of the humeral head can improve the contact between the humeral head and the articulating surface.

This overview is intended to provide an overview of subject matter of the present patent application. It is not intended to provide an exclusive or exhaustive explanation of the present subject matter. The present invention is defined in the independent claim, to which reference should now be made. Advantageous embodiments are set out in the sub claims. The detailed description is included to provide further information about the present patent application.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
Figure 1 is a perspective view of a humeral implant according to an example of the present disclosure.
Figure 2 is a schematic bottom view of a spherical humeral head according to an example of the present disclosure.
Figure 3 is a side cross-sectional view of the spherical humeral head depicted in Figure 2.
Figure 4 is a schematic bottom view of an oval humeral head according to an embodiment of the invention.
Figure 5 is a side cross-sectional view of the oval humeral head depicted in Figure 4.
Figure 6 is a side cross-sectional view of the oval humeral head depicted in Figure 4.

### DETAILED DESCRIPTION

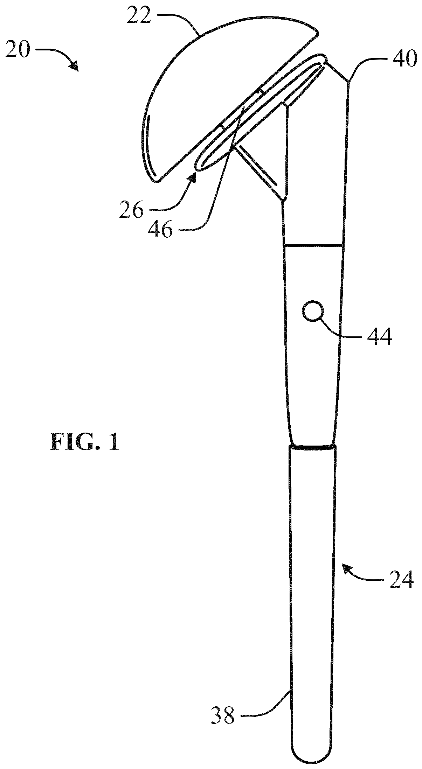

As depicted in FIG. 1, a humeral implant 20, according to an example of the present disclosure, can comprise a humeral head 22 and a humeral stem 24. An attachment assembly 26 can be provided for coupling the humeral head 22 to the humeral stem 24. In operation, the humeral stem 24 is insertable into the humerus to position the humeral head 22 such that the humeral head 22 approximates the natural humeral head. The humeral head 22 can engage the glenoid surface of the glenoid cavity. In an example, a glenoid implant can be mounted to the glenoid cavity to provide a glenoid surface.

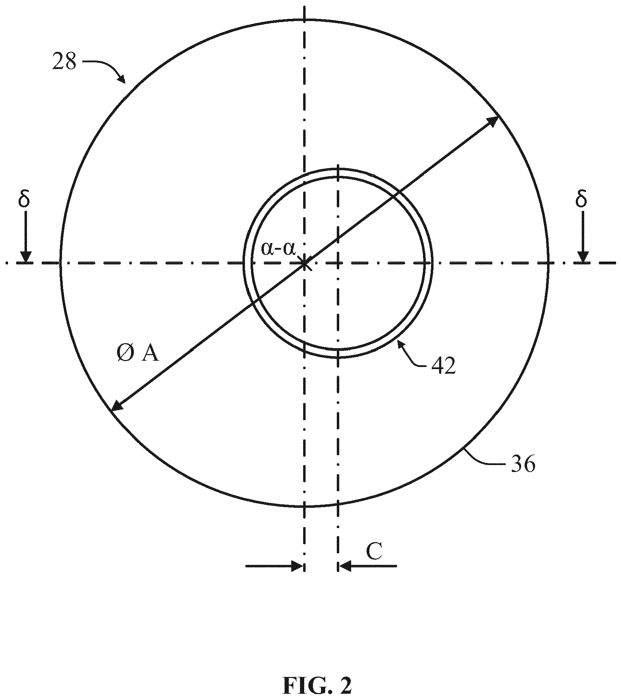

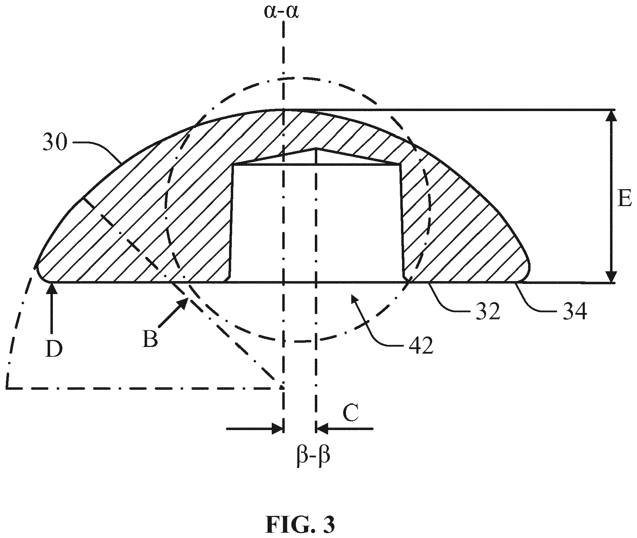

With reference to FIG. 2, in an example, the humeral head 22 can include a main body 28 defining a curved exterior surface 30 and a generally planar exterior surface 32. The main body 28 can further include an edge transition 34 (shown in FIG. 3) positioned between the curved exterior surface 30 and the generally planar exterior surface 32. In an example, the edge transition 34 can be equidistant from the main body axis α-α from an outer edge 36 of the main body 28. In at least one example, the distance of the edge transition 34 from the main body axis α-α is less than the distance from the outer edge 36 from the main body axis α-α. In this configuration, the outer edge 36 is curved as part of the curved exterior surface 30.

With reference again to FIG. 1, in an example, the humeral stem 24 can include a stem portion 38 and a mount portion 40. The stem portion 38 is insertable into humerus after the humeral head is resected. In at least one example, stem portion 38 can have a roughened surface for retaining the stem portion 38 for retaining the stem portion 38 within the humerus. The stem portion 38 can include at least one suture aperture 44 for suturing the stem portion 38 within the humerus. In an example, the mount portion 40 can be angled to the stem portion 38 to angle the humeral head 22 to simulate the natural angle of the humeral head.

As illustrated in FIGS. 1-3, in an example, the mount portion 40 of the humeral stem 24 can include a port 42 for receiving the attachment assembly 26 for attaching the humeral head 22 to the humeral stem 24. The port 42 can define a port axis β-β parallel to the main body central axis α-α. The attachment assembly 26 can include an attachment pin 46 attachable to the generally planar exterior surface 32 such that the attachment pin 46 extends from the generally planar exterior surface 32. The attachment pin 46 can be inserted into the port 42 to attach the humeral head 22 to the humeral stem 24.

As illustrated in FIGS. 2-3, the geometry of a spherically shaped humeral head 22 can be expressed by: a head footprint A; a curvature radius B; a port offset C; an edge transition position D; and a head height E.

As depicted in FIG. 2, the head footprint A can comprise the diameter of the main body 28 intersecting the main body axis α-α. In an example, the diameter of the main body 28 can be measured from opposing sides of the outer edge 36 of the main body 28 through the main body axis α-α along a radial axis δ-δ. In at least one example, the diameter of the main body 28 can be measured from opposing sides of the edge transition 34 of the main body 28 through the main body axis α-α, when the edge transition 34 is at the outer edge 36 of the main body 28.

As depicted in FIG. 3, the curvature radius B can comprise the radial distance from the curved exterior surface 30 to a center point of a sphere having a curvature corresponding to the curved exterior surface 30.

As depicted in FIGS. 2-3, the port offset C can comprise the distance between the main body axis α-α and the port axis β-β when the port 42 is offset from the center of the main body 28. In at least one example, the port 42 can be centered on the main body 28 such that the distance between the main body axis α-α and the port axis β-β is minimal.

As depicted in FIG. 3, the edge transition position D can comprise the distance from the main body axis α-α to the edge transition 34.

As further depicted in FIG. 3, the head height E can comprise the distance from the curved exterior surface 30 to the planer exterior surface 32. In an example, the head height E can comprise the distance between the curved exterior surface 30 and the planar exterior surface 28 at the main body axis α-α. In at least one example, the head height E can comprise the maximum distance between the curved exterior surface 30 and the planar exterior surface 28.

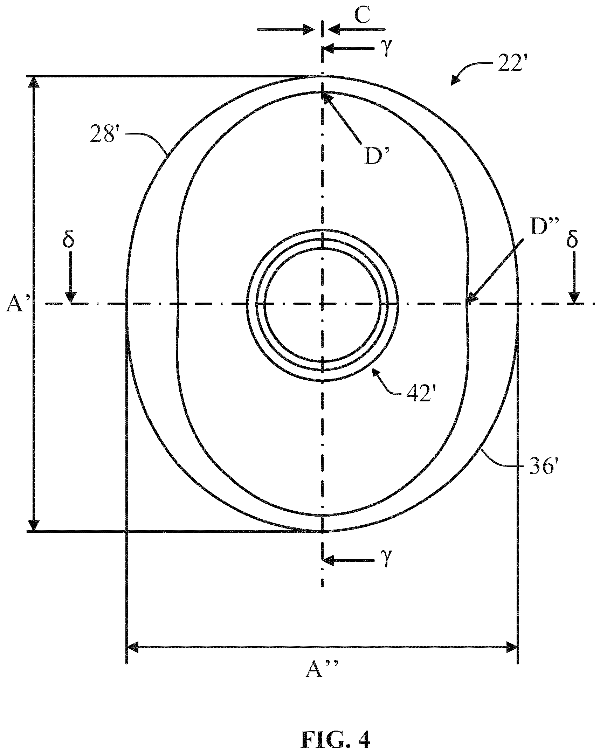

As illustrated in FIGS. 4-6, in an embodiment of the present invention, the geometry of an oval shaped humeral head 22' can be expressed by: a polar footprint A'; an equatorial footprint A"; a curvature radius B; a port offset C (not shown); a polar edge transition position D'; an equatorial edge transition position D"; and head height E'.

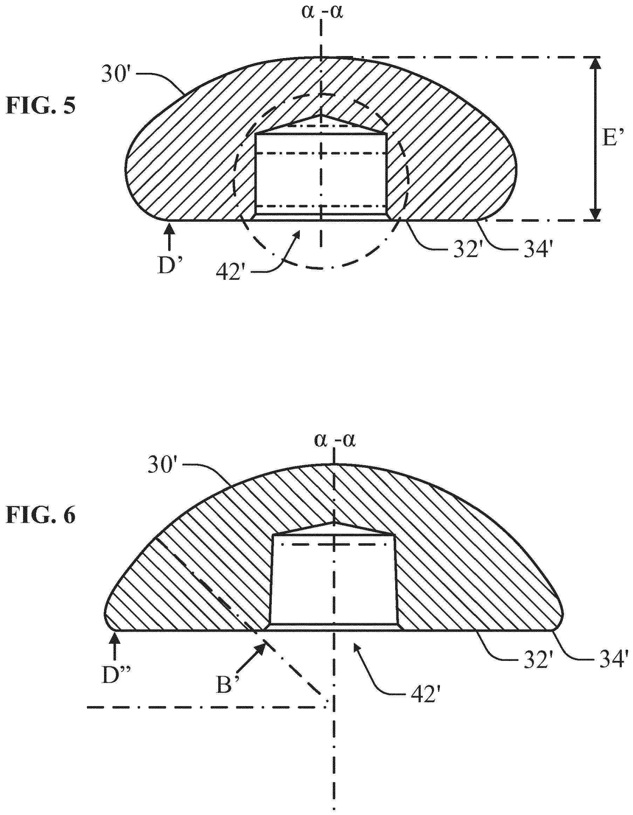

As depicted in FIG. 4, the humeral head 22 can include a polar axis γ-γ intersecting the main body axis α-α (FIG. 5), wherein the polar axis γ-γ is perpendicular to the main body axis α-α. The humeral head 22 can also include an equatorial axis δ-δ intersecting the main body axis α-α, wherein the equatorial axis δ-δ is perpendicular to the main body axis α-α and the polar axis γ-γ. In this configuration, the polar footprint A' can comprise the distance from opposing sides of the outer edge 36' of the main body 28' through the main body axis α-α and along the polar axis γ-γ. The equatorial footprint A" can comprise the distance from opposing sides of the outer edge 36' of the main body 28' through the main body axis α-α and along the equatorial axis δ-δ. In at least one example, the distance of the main body 28' for the polar footprint A' and the equatorial footprint A" can be measured from opposing sides of the edge transition 34' of the main body 28' through the main body axis α-α, when the edge transition 34' is at the outer edge 36' of the main body 28'.

As depicted in FIG. 6, the curvature radius B' can comprise the radial distance from the curved exterior surface 30' to a center point of a sphere having a curvature corresponding to the curved exterior surface 30'.

As depicted in FIG. 4, the port offset C can comprise the distance between the main body axis α-α and the port axis β-β when the port 42' is offset from the center of the main body 28'. In at least one example, the port 42' can be centered on the main body 28' such that the distance between the main body axis α-α and the port axis β-β is minimal.

As depicted in FIG. 5, the polar edge transition position D' can comprise the distance from the main body axis α-α to the edge transition 34' along the polar axis γ-γ. As depicted in FIG. 6, the equatorial edge transition position D" can comprise the distance from the main body axis α-α to the edge transition 34' along the equatorial axis δ-δ. In an example, the polar edge transition position D' can differ from the equatorial edge transition position D" such that the curvature of the main body 28' differs around the circumference of the main body 28'.

As depicted in FIG. 5, the head height E' can comprise the distance from the curved exterior surface 30' and the planer exterior surface 32'. In an example, the head height E can comprise the distance between the curved exterior surface 30' and the planar exterior surface 28' at the main body axis α-α. In at least one example, the head height E can comprise the maximum distance between the curved exterior surface 30' and the planar exterior surface 28'.

The above detailed description includes references to the accompanying drawings, which form a part of the detailed description. The drawings show, by way of illustration, specific embodiments in which the present subject matter can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any referenced documents, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other.

## Claims

1. A humeral implant, comprising:
an oval shaped humeral head (22') having a curved exterior surface (30') and a generally planar exterior surface (32'), the humeral head defining:
an outer edge (36');
a main body axis (α-α) extending transversely from the generally planar exterior surface (32') to the curved exterior surface (30'),
a polar axis (γ-γ) extending parallel to the planar exterior surface (32') from the outer edge (36') of the humeral head intersecting the main body axis (α-α),
an equatorial axis (δ-δ) extending parallel to the planar exterior surface (32') from the outer edge (36') of the humeral head intersecting the main body axis (α-α) and the polar axis (γ-γ); and
a polar edge transition (34') between the planar exterior surface (32') and the curved exterior surface (30'), wherein the polar edge transition (34') is positioned (D') at the outer edge (36') of the humeral head; and a humeral stem (24) insertable into a humerus, **characterised in that** an equatorial edge transition (34') is positioned (D") inward of the outer edge (36') of the humeral head and closer to the main body axis (α-α) than the polar edge transition (34').

2. The humeral implant of claim 1, the humeral head further comprising:
a port (42') for receiving an attachment assembly (26) for engaging the humeral stem (24).

3. The humeral implant of claim 2, wherein the port (42') defines a port axis (β-β) parallel to the main body axis (α-α).

4. The humeral implant of claim 2, wherein the main body axis (α-α) is centered in the humeral head (22'), and wherein the port (42') is offset from the main body axis.

5. The humeral implant of claim 1, wherein the humeral head (22') further comprises:
a polar diameter (A') defined along the polar axis (γ-γ); and
an equatorial diameter (A") defined along the equatorial axis (δ-δ);
wherein the polar diameter is different from the equatorial diameter.

6. The humeral implant of claim 5, wherein the polar diameter (A') is greater than the equatorial diameter (A").

7. The humeral implant of claim 1, wherein the humeral stem (24) further comprises:
a stem portion (38) insertable into the humerus; and
a mount portion (40) for receiving the humeral head.

8. The humeral implant of claim 7, wherein the mount portion (40) is offset from the stem portion (38) such that, upon engagement of the humeral head (22') with the humeral stem (24), the humeral head (22') is angled relative to the stem portion (38).

9. The humeral implant of claim 7, wherein the stem portion (38) is roughened to improve retention of the stem portion within the humerus.

## Patentansprüche

1. Humerusimplantat, aufweisend:
eine oval geformten Humeruskopf (22') mit einer gekrümmten Außenfläche (30') und einer im Allgemeinen planaren Außenfläche (32'), wobei der Humeruskopf definiert:
eine Außenkante (36'):
eine Hauptkörperachse (α-α), die sich in Querrichtung von der im Allgemeinen planaren Außenfläche (32') zu der gekrümmten Außenfläche (30') erstreckt,
eine Polachse (γ-γ), die sich parallel zu der planaren Außenfläche (32') von der Außenkante (36') des Humeruskopfs erstreckt und die Hauptkörperachse (α-α) überschneidet,
eine Äquatorialachse (δ-δ), die sich parallel zu der planaren Außenfläche (32') von der Außenkante (36') des Humeruskopfs erstreckt und die Hauptkörperachse (α-α) und die Polachse (γ-γ) überschneidet; und
einen Polkantenübergang (34') zwischen der planaren Außenfläche (32') und der gekrümmten Außenfläche (30'), wobei der Polkantenübergang (34') an der Außenkante (36') des Humeruskopfs positioniert (D') ist; und den in einen Humerus einsetzbaren Humerusschaft (24), **dadurch gekennzeichnet, dass** ein Äquatorialkantenübergang (34') einwärts der Außenkante (36') des Humeruskopfs und näher an der Hauptkörperachse (α-α) als dem Polkantenübergang (34') positioniert (D") ist.

2. Humerusimplantat nach Anspruch 1, wobei der Humeruskopf ferner aufweist:
eine Öffnung (42') zum Aufnehmen einer Befestigungsanordnung (26) zum Eingreifen in den Humerusschaft (24).

3. Humerusimplantat nach Anspruch 2, wobei die Öffnung (42') eine Öffnungsachse (β-β) definiert, die parallel zu der Hauptkörperachse (α-α) verläuft.

4. Humerusimplantat nach Anspruch 2, wobei die Hauptkörperachse (α-α) in dem Humeruskopf (22') mittig angeordnet ist, und wobei die Öffnung (42') von der Hauptkörperachse versetzt ist.

5. Humerusimplantat nach Anspruch 1, wobei der Humeruskopf (22') ferner aufweist:
einen Poldurchmesser (A'), der entlang der Polachse (γ-γ) definiert ist; und
einen Äquatorialdurchmesser (A"), der entlang der Äquatorialachse (δ-δ) definiert ist;
wobei der Poldurchmesser von dem Äquatorialdurchmesser verschieden ist.

6. Humerusimplantat nach Anspruch 5, wobei der Poldurchmesser (A') größer als der Äquatorialdurchmesser (A") ist.

7. Humerusimplantat nach Anspruch 1, wobei der Humerusschaft (24) ferner aufweist:
einen Schaftabschnitt (38), der in den Humerus einsetzbar ist; und
einen Befestigungsabschnitt (40) zum Aufnehmen des Humeruskopfs.

8. Humerusimplantat nach Anspruch 7, wobei der Befestigungsabschnitt (40) von dem Schaftabschnitt (38) versetzt ist, sodass bei Eingreifen des Humeruskopfs (22') in den Humerusschaft (24) der Humeruskopf (22') relativ zu dem Schaftabschnitt (38) angewinkelt ist.

9. Humerusimplantat nach Anspruch 7, wobei der Schaftabschnitt (38) aufgeraut ist, um die Bindung des Schaftabschnitts in dem Humerus zu verbessern.

## Revendications

1. Implant huméral, comprenant :
une tête humérale de forme ovale (22') possédant une surface externe incurvée (30') et une surface externe généralement plane (32'), la tête humérale définissant :
un bord externe (36') ;
un axe de corps principal (α-α) s'étendant transversalement de la surface externe généralement plane (32') à la surface externe incurvée (30'),
un axe polaire (γ-γ) s'étendant parallèle à la surface externe plane (32') depuis le bord externe (36') de la tête humérale coupant l'axe de corps principal (α-α),
un axe équatorial (δ-δ) s'étendant parallèle à la surface externe plane (32') depuis le bord externe (36') de la tête humérale coupant l'axe de corps principal (α-α) et l'axe polaire (γ-γ) ; et
une transition de bord polaire (34') entre la surface externe plane (32') et la surface externe incurvée (30'), ladite transition de bord polaire (34') étant positionnée (D') au niveau du bord externe (36') de la tête humérale ; et
une tige humérale (24) insérable dans un humérus,
**caractérisé en ce qu'**une transition de bord équatoriale (34') est positionnée (D") vers l'intérieur du bord externe (36') de la tête humérale et plus proche de l'axe de corps principal (α-α) que la transition de bord polaire (34').

2. Implant huméral selon la revendication 1, ladite tête humérale comprenant en outre :
un orifice (42') pour recevoir un ensemble de fixation (26) pour engager la tige humérale (24).

3. Implant huméral selon la revendication 2, ledit orifice (42') définissant un axe d'orifice (β-β) parallèle à l'axe de corps principal (α-α).

4. Implant huméral selon la revendication 2, ledit axe de corps principal (α-α) étant centré dans la tête humérale (22'), et ledit orifice (42') étant décalé par rapport à l'axe de corps principal.

5. Implant huméral selon la revendication 1, ladite tête humérale (22') comprenant en outre :
un diamètre polaire (A') défini le long de l'axe polaire (γ-γ) ; et
un diamètre équatorial (A") défini le long de l'axe équatorial (δ-δ) ;
ledit diamètre polaire étant différent du diamètre équatorial.

6. Implant huméral selon la revendication 5, ledit diamètre polaire (A') étant supérieur au diamètre équatorial (A").

7. Implant huméral selon la revendication 1, ladite tige humérale (24) comprenant en outre :
une partie tige (38) insérable dans l'humérus ; et
une partie de montage (40) pour recevoir la tête humérale.

8. Implant huméral selon la revendication 7, ladite partie de montage (40) étant décalée par rapport à la partie tige (38) de sorte que, lors de l'engagement de la tête humérale (22') avec la tige humérale (24), la tête humérale (22') est inclinée par rapport à la partie tige (38).

9. Implant huméral selon la revendication 7, ladite partie tige (38) étant rendue rugueuse pour améliorer la rétention de la partie tige dans l'humérus.
